Europäisches Patentamt

European Patent Office

Office européen des brevets

(11) Publication number: **0 255 320**
**A2**

# EUROPEAN PATENT APPLICATION

(21) Application number: **87306608.8**

(22) Date of filing: **27.07.87**

(51) Int. Cl.⁴: **C12N 15/00** , **C12N 5/00** , **C12P 21/02**

Claims for the following Contracting States: AT + ES.

The microorganism(s) has (have) been deposited with American Type Culture Collection, Rockville, Maryland under number(s) ATCC 67162.

(30) Priority: **28.07.86 US 891259**

(43) Date of publication of application: **03.02.88 Bulletin 88/05**

(84) Designated Contracting States:
**AT BE CH DE ES FR GB GR IT LI LU NL SE**

(71) Applicant: **Integrated Genetics, Inc.**
**31 New York Avenue**
**Framingham Massachusetts 01701(US)**

(72) Inventor: **Hendricks, Marvin B.**
**21 Perry H. Henderson Drive**
**Framingham Massachusetts 01701(US)**
Inventor: **Banker, Michael J.**
**38 Prospect Street**
**Hopedale Massachusetts 01747(US)**

(74) Representative: **Sheard, Andrew Gregory et al**
**Kilburn & Strode 30, John Street**
**London WC1N 2DD(GB)**

(54) **Production of proteins in myeloma cells.**

(57) Mammalian protein expression vectors, containing a non-immunoglobulin gene encoding a desired protein, a promoter sequence chosen from an Ig promoter, a metallothionein promoter or a cytomegalovirus promoter, and an enhancer sequence from an Ig gene or a cytomegalovirus, are transfected into myeloma cells for the production of the desired protein.

EP 0 255 320 A2

## PRODUCTION OF PROTEINS IN MYELOMA CELLS

This invention relates to systems for the expression of proteins in mammalian cells.

Immunoglobulin (Ig) genes can be expressed at high levels when introduced into myeloma cells. Expression of an Ig gene in myeloma cells is facilitated by the presence of the Ig gene enhancer and promoter regions. For example, Kudo et al. (EP-A-0146743, Teijin Limited) describe the transformation of mouse myeloma cells with a vector containing a human IgG gene, along with its enhancer and promoter sequences, and the expression of this Ig gene in the cell.

In general, the invention features mammalian (e.g., human) myeloma cells transformed with vectors capable of expressing desired non-Ig proteins in the myeloma cells. Preferred vectors of the invention include a gene encoding the desired protein, a promoter sequence chosen from an Ig promoter, a metallothionein promoter, or a cytomegalovirus (CMV) promoter, and an enhancer sequence derived from an Ig gene or a CMV gene. The enhancer sequence stimulates transcription initiation from the promoter, thereby increasing production of the desired protein.

In preferred embodiments the vector also contains Ig gene untranslated regions ("splice DNA" or "introns") and a transcription termination signal, such as the polyadenylation (Poly A) signal derived from SV40 or Ig DNA. Preferably the enhancer is positioned in the vector upstream from the Ig promoter or within the splice DNA. The desired protein to be expressed can be, for example, t-PA, hepatitis B surface antigen, or a fertility hormone.

Other features and advantages of the invention will be apparent from the following description of the preferred embodiments and from the claims.

The drawings will first briefly be described.

### Drawings

Fig. 1 is a diagrammatic representation of the alteration of a Ddel site to a Xhol site in pLT31, yielding pLT31X; P = PvuII, R1 = EcoR1, K = KpnI, B = BamH1;

Figs. 2a and 2b are diagrammatic representations of the construction of selectable plasmids useful in the invention;

Figs. 3 a-d are diagrammatic representations of expression vectors of the invention;

Figs. 4 a-d are diagrammatic representations of expression vectors having foreign genes inserted;

Fig. 5 is a diagrammatic representation of the substitution of an MT-1 promoter for an Ig promoter in an expression vector;

Fig. 6 is a diagrammatic representation of HCMV AD169; and

Fig. 7 is a diagrammatic representation of the insertion of a CMV promoter and enhancer into plasmid B.

### Structure

Components of the invention will now be described in more detail.

### Enhancer

Enhancers are DNA sequences that stimulate the level of transcription from a particular promoter sequence. Enhancers of the invention can be derived from an Ig gene or a cytomegalovirus, as described by Weber et al. (1984, Cell 36:983), and Boshart et al. (1985, Cell 41:521). The enhancer may be positioned at any part of the vector of the invention provided it is not located within the structural DNA encoding the desired protein. An enhancer sequence is usually positioned within approximately 10kb of the promoter, preferably 30-400 bp upstream of the promoter sequence which it is to stimulate. Examples of suitable enhancer sequences are those derived from pLT31 (Stafford et al., 1983, Nature 306:77) and $pV_HC_K$ (Sharon et al., 1984 Nature 309:364). Such enhancers are capable of stimulating heterologous promoters, i.e., promoters not naturally associated with the enhancer.

Enhancers are thought to act as entry sites for RNA polymerase or to act synergistically with promoters to enhance transcription. Enhancers cause more RNA polymerase II molecules to initiate transcription, thus increasing the density of polymerase along the DNA and consequently the rate of transcription of the gene.

### Promoter

Promoters are DNA sequences which control the level of transcription of a gene. The promoter must be positioned in the vector proximally upstream from the structural gene in order to control transcription of that gene. Promoters useful in the invention can be derived from any Ig gene, a cytomegalovirus, or a mammalian metallothionein (MT) gene. For example, the mouse metallothionein gene promoter is inducible by cadmium and zinc and is relatively strong, thus allowing for selective induction.

## Desired gene

Any gene which encodes a desired protein may be used. The gene may or may not contain untranslated regions (introns) and preferably contains DNA encoding the natural leader sequence of the protein. Examples include t-PA, fertility hormones and hepatitis B antigens, as described in copending applications U.S.S.Nos. 656,770, 548,228, and 570,940, assigned to the same assignee as this application, hereby incorporated by reference.

## Poly A signal

A poly A signal specifies cleavage of precursor mRNA within 30 bases of the poly A site and the addition of the poly A tail, which stabilizes the mRNA transcript. Almost all eukaryotic genes have such signals; thus, poly A signal sequences for the vectors of the invention can be obtained from almost any eukaryotic gene. In general, a poly A signal has a core sequence of AAATAA, as exemplified by the poly A signal of SV40.

## Myeloma cell

Any myeloma cell (cancerous B lymphocyte) which can be cultured to produce antibodies and transfected by plasmid vectors is suitable. Examples are cell lines such as the commercially available (American Type Culture Collection) Sp 2.0 cells (Shulman et al., 1978, Nature 276:269) and MPC-11 cells (Laskov et al., 1970, J. Exp. Med. 131:515), or J558L cells (Oi et al., 1983, Proc. Nat. Acad. Sci. USA 80, 825). J558L cells are particularly suitable for plasmids having the MT promoter sequence.

## Particular Vectors

### pLT31

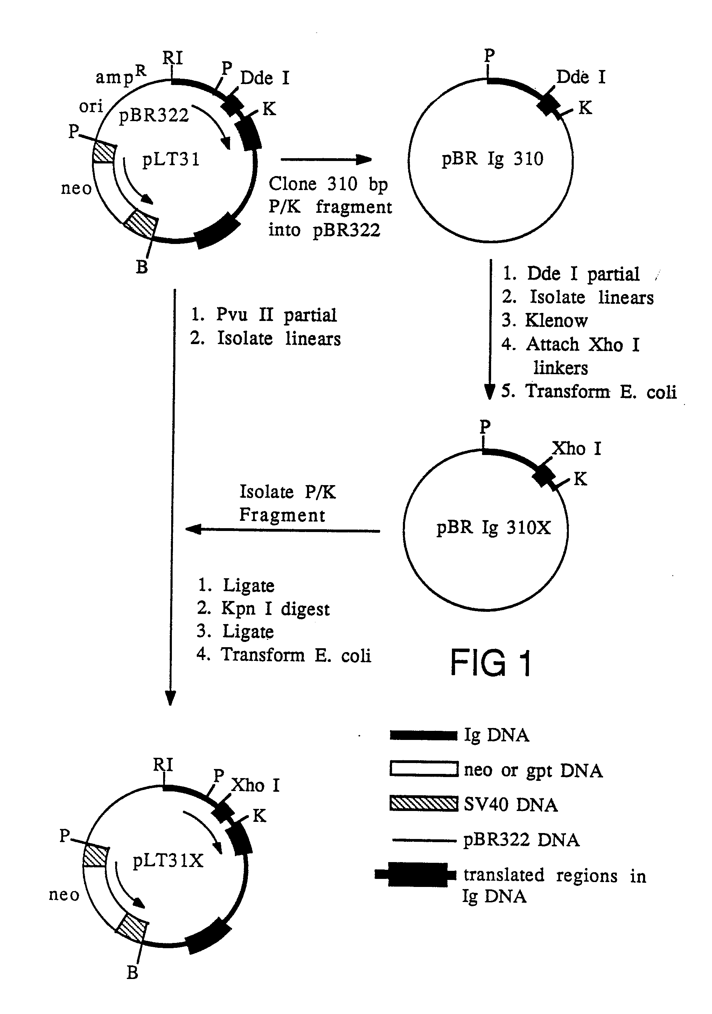

In the following examples all vectors were derived from pLT31 (Stafford et al., supra). Referring to Fig. 1, pLT31 (obtained from Cary Queen, N.I.H., Bethesda, MD) includes a 7kb genomic fragment from mouse myeloma MOPC41 (thickened line) containing the Ig Kappa light chain gene including two introns (shown by the region containing three solid blocks), and DNA sequences encoding an Ig promoter (between the Pvull and Ddel site). pLT31 also includes SV40 promoter and poly A sequences (shaded blocks), the neo gene, and a pBR322 origin and selectable marker (amp^R). The arrows represent the direction of transcription from the Ig and SV40 promoters.

## Construction of pLT31X

Referring to Fig. 1, a Xhol site was substituted for a Ddel site in pLT31 to give pLT31X. pLT31 was restricted with Pvull and Kpnl, and the resulting 310 bp fragment was ligated into similarly-cut pBR322 to give pBRIg310. This plasmid was then cut partially with Ddel, linear molecules were isolated and treated with Klenow, and Xhol were linkers attached. After transformation into E. coli , some of the resulting colonies contained pBRIg310X, having a Xhol site in place of the Ddel site. The Pvu II/Kpnl fragment from pBRIg310X was then isolated and ligated into pLT31 which had been partially cut with Pvull. This linear molecule was cut with Kpnl and then ligated together. After transformation of E. coli some of the resulting colonies contained pLT31X.

## Selectable plasmids

Selectable plasmids are used according to this invention to facilitate the isolation of transformants containing the non-selectable expression plasmids of the invention. Transfections are performed using the selectable plasmid together with a large molar excess of an expression plasmid containing the gene for the desired protein; transfected cells are selected as those exhibiting the selectable marker trait. The two types of plasmids are simultaneously incorporated into transfected cells 50-70% of the time.

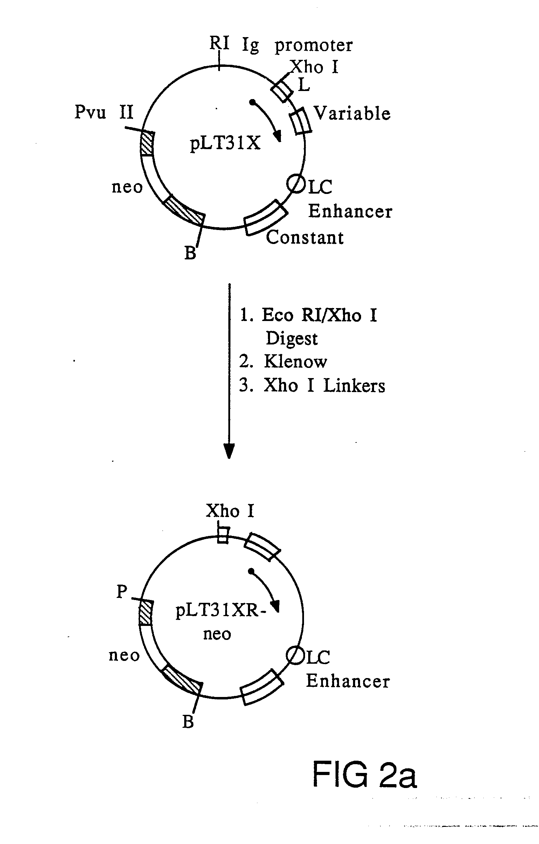

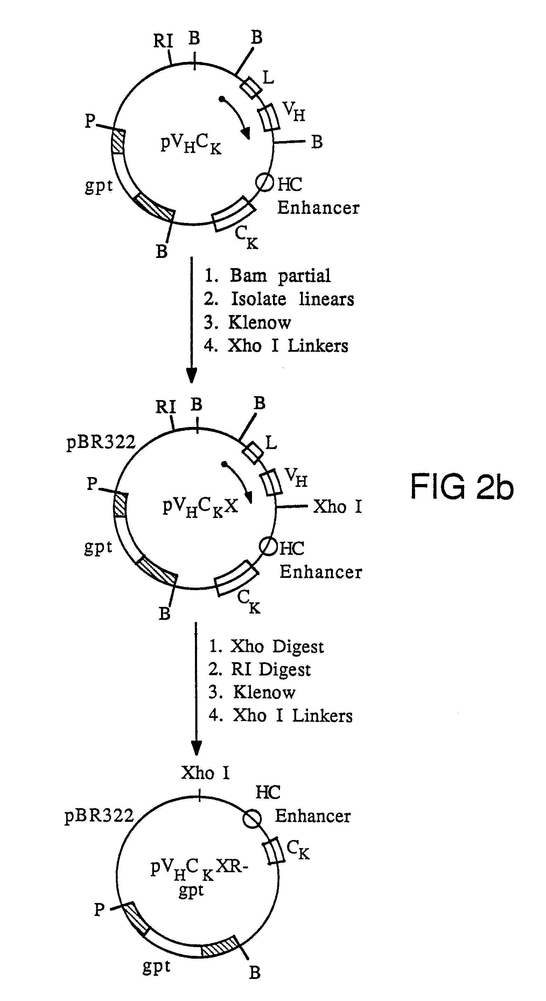

Selectable plasmids contain a selectable marker gene, such as neo (resistance to the antibiotics neomycin and G418) or gpt (resistance to mycophenolic acid), which is expressed in myeloma cell lines. Examples of such plasmids are pSV2neo and pSV2gpt (American Type Culture Collection accession numbers 37,149 and 37,145, respectively; Southern et al., 1982, J. Mol. & Applied Gen., 1:327; Mulligan et al., 1980, Science 209: 1422). Other selectable plasmids which are suitable are constructed, for example, from pLT31X and pV_HC _K. The Ig promoter sequences are removed from these plasmids so that expression of the Ig gene is prevented; the enhancer sequences remain on the plasmids. These enhancer sequences improve the frequency of cell transformation to drug resistance. As shown in Fig. 2a, selectable plasmid pLT31XR-neo, having the neo marker, is derived from pLT31X by digestion with EcoRI and XhoI, Klenow treatment, addition of XhoI linkers, and ligation, thus removing the Ig promoter. As shown in Fig. 2b, a gpt selectable plasmid pV$_H$C$_K$XR-gpt is derived from PV$_H$C$_K$ by partial BamHI digestion, isolation of linear molecules, Klenow treatment, and ligation of XhoI linkers. After transformation of this ligation mixture into E. coli , some of the resulting colonies contained pV$_H$C$_K$ X having an XhoI site in the 2nd Ig intron. The Ig promoter region was removed from pV$_H$C$_K$X by digestion with XhoI and EcoRI, treatment with Klenow, and ligation of XhoI linkers, yielding pV$_H$C$_K$XR-gpt.

Construction of Expression Vectors

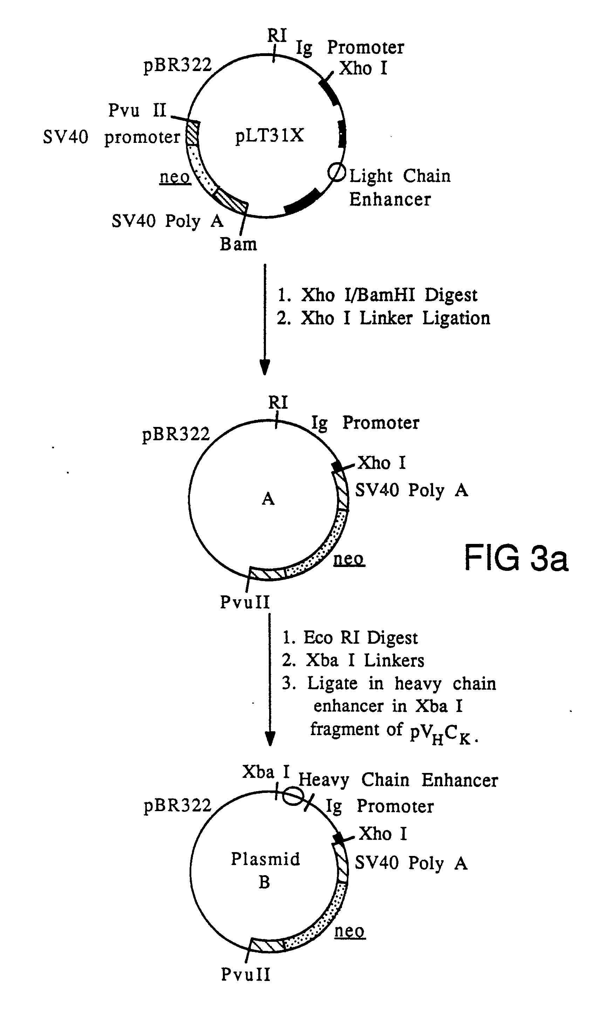

A vector of the invention, which contains no Ig structural gene or splice DNA, was constructed from pLT31X by digestion with XhoI and BamH1, followed by ligation of XhoI linkers, as shown in Fig. 3a. The resulting plasmid A was then restricted with EcoR1, Xbal linkers were attached, and the Xba I heavy chain enhancer fragment from pV$_H$C$_K$was inserted, yielding plasmid B. DNA encoding a desired protein can be readily inserted into plasmid B by cutting with XhoI and ligating the DNA's together. This places the inserted gene under the control of the Ig promoter and the pV$_H$C$_K$ enhancer.

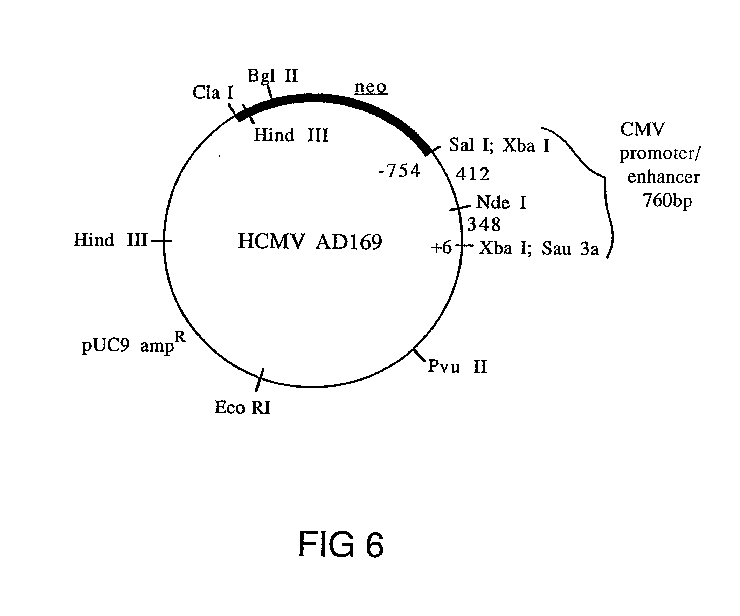

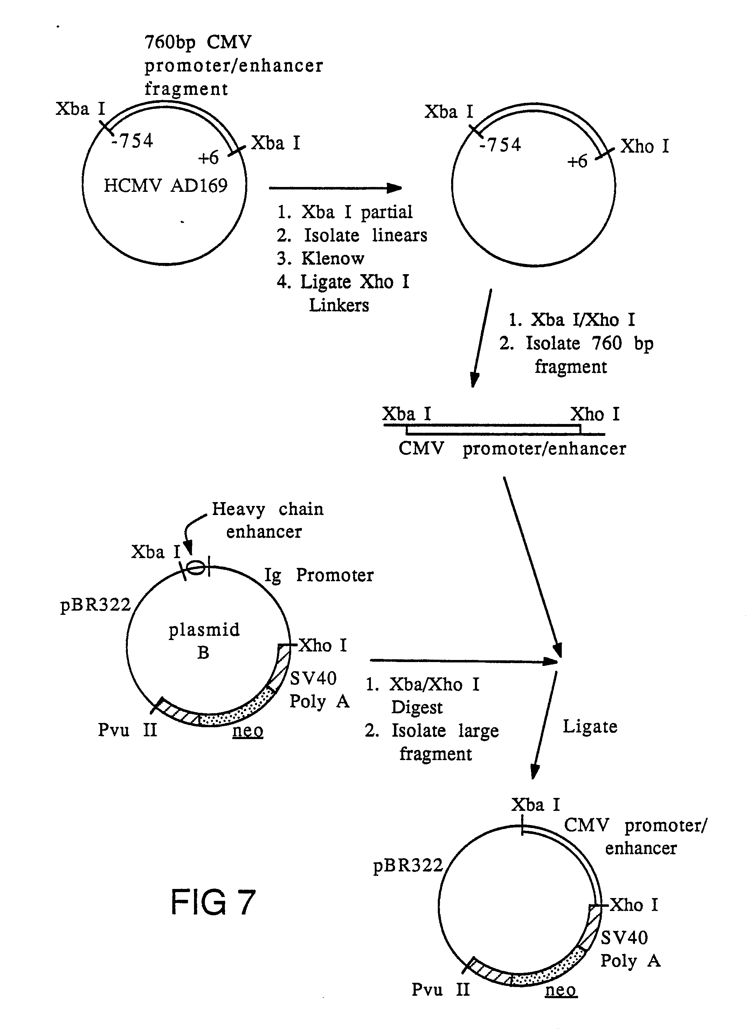

The Ig promoter can be replaced in plasmid B with the 0.76kb human cytomegalovirus enhancer/promoter fragment. Referring to Fig. 6, plasmid HCMV AD169 (obtained from Deborah Spector, University of California, San Diego) includes a 760 bp Xbal fragment containing the human CMV enhancer and promoter. Referring to Fig. 7, the Xbal site at the position labelled +6 in HCMV AD169 was converted to an XhoI site by attaching XhoI linkers. HCMV AD169 was partially digested with Xbal; linear molecules were isolated and Klenow treated; XhoI linkers were ligated onto these treated molecules; and the ligation mixture was transformed into E. coli. Some of the resulting colonies contained plasmids with a XhoI site in the desired location. These plasmids were digested with Xba I and XhoI, and the 760 bp Xbal/XhoI ended fragment was then isolated and ligated into the solated large Xbal/XhoI fragment of plasmid B, thus replacing the Ig enhancer/promoter region with the CMV enhancer/promoter.

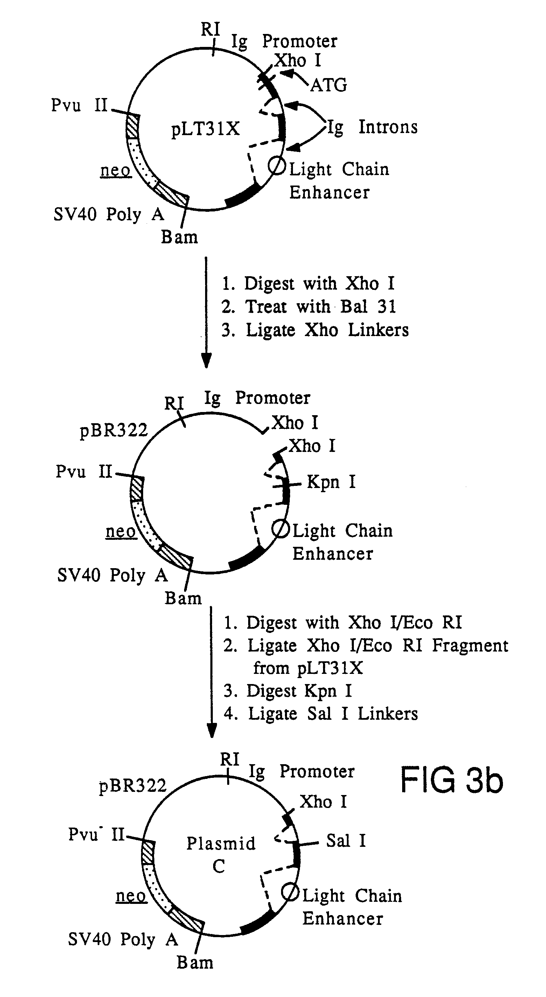

Another vector of the invention, with the cloning site for the foreign gene downstream of the first Ig gene intron, was constructed as follows. Referring to Fig. 3b, pLT31X was digested with XhoI and then treated with Bal31 until enough DNA had been digested to remove the initiator ATG of the Ig gene. This Bal 3l treatment was performed as follows. Aliquots were taken from the Bal3l/pTL3lX treatment mixture, and the Bal3l enzyme was immediately killed. Samples from the aliquots were cut with KpnI, and the length of the KpnI/XhoI fragment in each sample was determined. XhoI linkers were then ligated to the end of the Bal31-treated pLT31X aliquot in which the KpnI/XhoI fragment had been reduced from 180bp to approximately 150 bp. The sequences originally upstream of the XhoI site were replaced by ligating the XhoI-Eco R1 fragment of untreated pLT31X into the Bal31-treated vector. The resulting vector was then digested with KpnI, and SalI linkers were ligated into this site, yielding plasmid C (Fig. 3b). A SalI DNA fragment encoding the desired protein then can be inserted into this vector. The inserted foreign DNA is under the control of the Ig promoter and 3′ enhancer sequences.

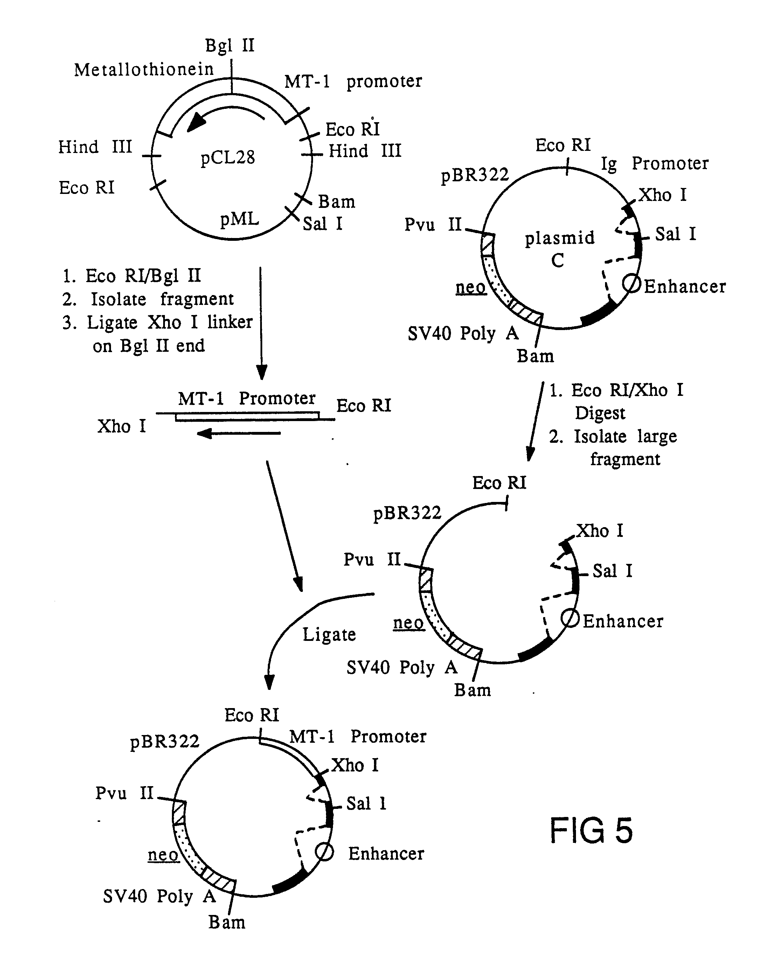

As an example of the use of alternate promoters, the Ig promoter in plasmid C can be replaced with the 1.9 kb metallothionein promoter fragment (Searle et al., (1984), Mol. and Cellular Biol. 4, 1221). Referring to Fig. 5, the MT-1 promoter fragment is isolated by EcoR1-BglII digestion of plasmid pCL28 (described, as pJYmmT(E), in Hamer et al., (1983) J. Mol. Applied Gen. 1, 273). XhoI linkers are then attached to the BglII end of the MT-1 fragment, and ligated to the large EcoR1/XhoI fragment of plasmid C.

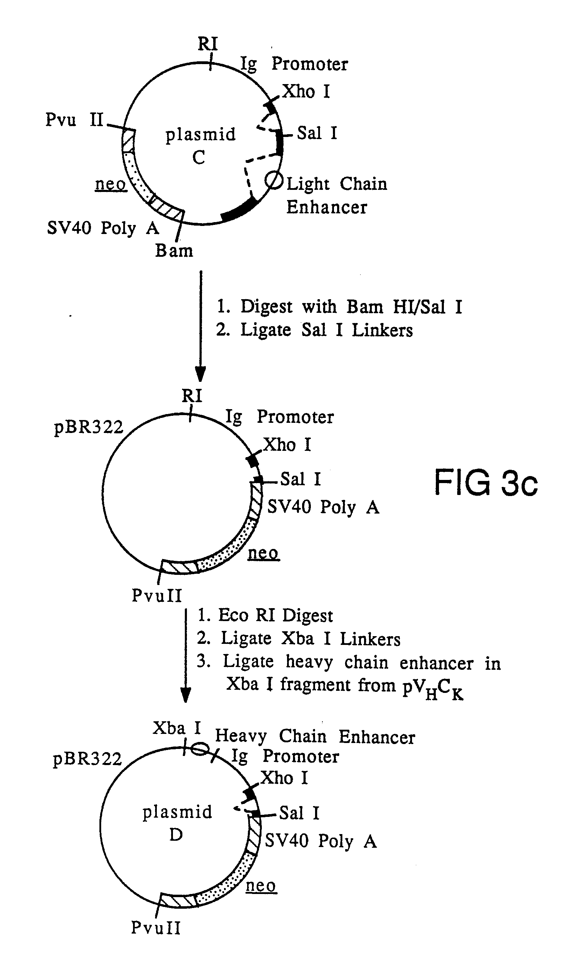

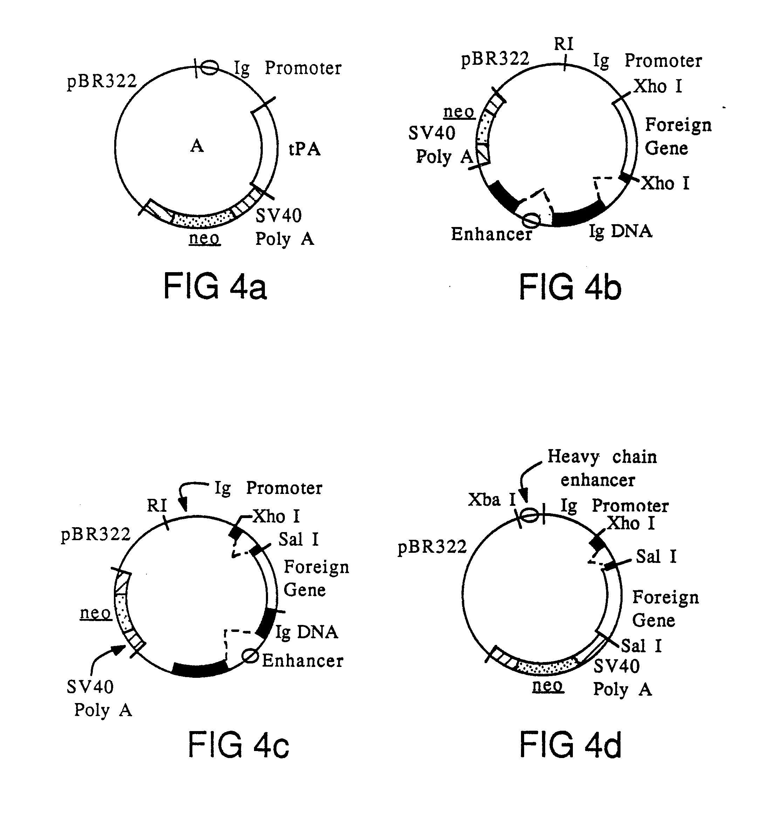

A vector having 5′ splice DNA upstream of the inserted foreign DNA (Fig. 4d) but lacking Ig gene coding sequences downstream of the cloning site was constructed as follows. Referring to Fig. 3c, plasmid C described above was digested with SalI and BamH1 to remove the downstream SalI/BamI Ig fragment, SalI linkers were added, and the SalI ends were ligated together. In this manner, the SV40 Poly A sequence is positioned just downstream from the cloning site. The EcoR1 site in the vector was then changed to an Xbal site by attaching Xbal linkers. An Ig heavy chain enhancer contained in an Xbal fragment of pV$_H$C$_K$ then was cloned into this new Xbal site, giving plasmid D (Fig. 3c). The desired foreign gene to be expressed is inserted into the SalI site of this plasmid.

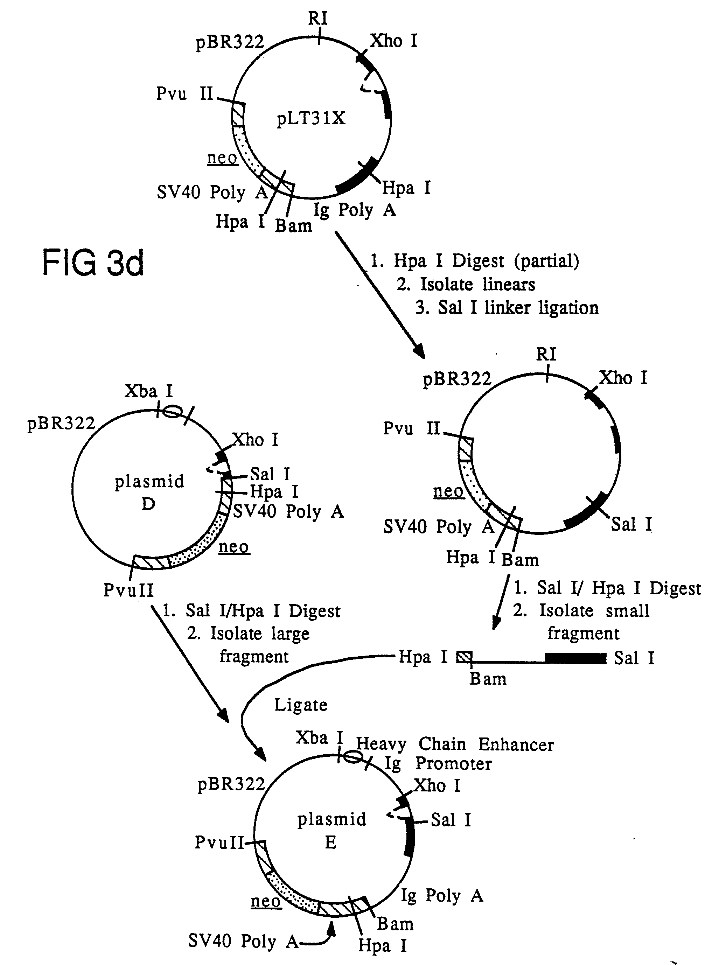

A vector similar to plasmid D but having the Ig Poly A sequence downstream of the cloning site, in place of the SV40 poly A sequences, was constructed as follows. Referring to Fig. 3d, pLT31X was partially digested with HpaI, linear molecules were isolated, and SalI linkers were ligated to the linear molecules. The resulting molecules having a Sall site in the protein coding sequences of the Ig constant region were digested with Hpal and Sall and the appropriate Hpal/Sall fragment was isolated. This fragment was ligated into a Sall/Hpal digest of plasmid D, yielding plasmid E.

## Insertion of Foreign Genes into the Vectors

Referring to Fig. 3a, above, plasmid B can be used to accept a desired foreign gene. For example, the Sall t-PA fragment (described in copending application U.S.S.N. 656,770, id.), containing a gene encoding the protein t-PA, was isolated and ligated into Xhol treated plasmid B. The resulting plasmid C is able to express large amounts of t-PA in myeloma cells (Fig. 4a).

Plasmid pLT31X itself (Fig. 1) can be used directly to efficiently express desired genes in myeloma cells, if the inserted gene is less than 400 base pairs in length. The vector is cut with Xho I, and the DNA encoding the desired protein is ligated directly into Ig gene 5' non-coding sequences (Fig. 4b). The Ig poly A termination signal serves as a termination signal for the inserted gene, and the 3' enhancer region increases transcription from the Ig promoter. Alternatively, the MT promoter can be used in place of the Ig promoter.

Referring to Fig. 4c, DNA encoding a desired protein is preferably inserted into the Sall site at the 5' end of the Ig gene of plasmid C, just downstream of the first Ig untranslated region. This vector also contains no interfering ATG codons upstream from the inserted gene of interest, and the vector expresses the desired protein at a higher rate than if the gene is inserted upstream from the first intron. The efficiency of splicing of the Ig 5' intron is unperturbed by these procedures. In any of the vectors described the MT promoter may be used in lieu of the Ig promoter, and the Ig poly A terminator sequence can be used as a terminator sequence for inserted genes.

A foreign gene can be inserted in the Sall cloning site of plasmid D, and the inserted gene is then under the control of the Ig promoter and upstream $pV_HC_K$ enhancer (Fig. 4d). In this vector the SV40 poly A termination sequence serves as terminator signal for inserted genes. This vector and plasmid E, its counterpart containing the Ig poly A signal, have proved to be the most efficient expression vectors of the ones described, as the heavy chain enhancer gives particularly strong stimulation of the Ig promoter.

The above examples are not meant to be exhaustive of the possible arrangements of promoter and enhancer sequences, as other equivalent arrangements are possible, and the invention is not limited to these examples. For example, the promoter and enhancer sequences may be replaced by those from the E1 gene of human cytomegalovirus. Such a substitution would allow full expression of multiple gene copies in myeloma cell lines where tissue-specific transcriptional factors might be limiting.

In the above examples all the vectors contain neo sequences. The neo sequences must be removed to achieve high copy number in the transfection of these vectors into myeloma cells. For plasmids B and D, the neo gene is removed by digestion with Ncol and Ndel, addition of Ncol linkers, and religation. For plasmid C, the neo sequences are removed by digestion with BamH1 and Nde I, addition of BamH1 linkers, and religation. For plasmid E, the neo sequences are removed by digestion with Hpal and Ndel, addition of HindIII linkers, and religation.

## Use

The vectors of the invention are transfected into myeloma cells by conventional techniques, e.g., electroporation (Potter et al., 1984, Proc. Nat. Acad. Sci. U.S.A. 81:7161). The transfection by electroporation preferably utilizes a two step process involving the introduction, by non-ligated co-transfer, of the desired plasmid in conjunction with two different selectable plasmids. In the first step the desired expression vector, containing the foreign gene to be expressed, is introduced into the myeloma cell with pLT31XR-neo as the selectable plasmid; the second step involves similar co-transfer, using $pV_HC_KXR$-gpt as the selectable plasmid. In this case, a high-producing cell line derived from neo selection is used as the host cell. Prior to co-transfer, all plasmids are linearized by restriction at a non-essential site. A 1:100 a ratio of selectable plasmid to expression vector at each step yields transfectants with multiple copies of the desired gene.

The transfected myeloma cells can be grown in commercially available hollow fiber or encapsulation culture systems to yield cell densities of greater than $10^7$ cells/cm³, preferably more than $10^8$ cells/cm³. Desired protein is isolated and purified by standard procedures.

## Deposits

E. coli cells containing plasmid D were deposited on July 24, 1986 with the American Type Culture Collection, Rockville, Maryland, and assigned ATCC accession number 67162.

Other embodiments are within the following claims.

## Claims

1. A mammalian myeloma cell transformed with a vector comprising:

a) a gene encoding a desired non-Ig protein;

b) a promoter sequence chosen from the promoter sequence of an Ig gene, a mammalian metallothionein gene, or a cytomegalovirus, positioned in said vector to effect transcription of said desired gene; and

c) an Ig enhancer sequence or a cytomegalovirus enhancer sequence capable of enhancing the expression of said desired gene in said myeloma cell by effecting transcription from said promoter.

2. A myeloma cell as claimed in claim 1 in which said vector further contains a DNA sequence which causes transcription termination and poly A addition.

3. A myeloma cell as claimed in claim 2 wherein said sequence is an SV40 poly A signal sequence or an Ig poly A signal sequence.

4. A myeloma cell as claimed in claim 1, 2 or 3 wherein said enhancer is positioned in said vector upstream from said promoter and within 1000 base pairs of said promoter.

5. A myeloma cell as claimed in any one of claims 1 to 4, wherein said vector contains Ig gene untranslated regions and said enchancer is positioned within one of said regions.

6. A myeloma cell as claimed in any one of claims 1 to 5, wherein said protein is t-PA, hepatitis B surface antigen, or a fertility hormone.

7. A myeloma cell as claimed in any one of claims 1 to 6, wherein said promoter sequence is an Ig light chain promoter, and said Ig enhancer is an Ig heavy chain enhancer.

8. A myeloma cell as claimed in any one of claims 1 to 7, wherein said promoter sequence is an Ig kappa light chain promoter.

9. A myeloma cell as claimed in claim 7 or 8, wherein said vector further comprises an Ig poly A signal sequence.

10. A method for producing a desired non-Ig protein in a myeloma cell, comprising culturing a myeloma cell as claimed in any one of claims 1 to 9 and isolating said desired non-Ig protein from said cell or its medium.

11. A vector comprising

a) a gene encoding a desired non-Ig protein;

b) a promoter sequence chosen from the promoter sequence of an Ig gene, a mammalian metallothionein gene, or a cytomegalovirus, positioned in said vector to effect transcription of said desired gene; and

c) an Ig enhancer sequence or a cytomegalovirus enhancer sequence capable of enhancing the expression of said desired gene in a myeloma cell by effecting transcription from said promoter.

Claims for the following contracting states: AT and ES

1. A process for the preparation of a transformed mammalian myeloma cell, the process comprising transforming a mammalian myeloma cell with a vector comprising:

a) a gene encoding a desired non-Ig protein;

b) a promoter sequence chosen from the promoter sequence of an Ig gene, a mammalian metallothionein gene, or a cytomegalovirus, positioned in said vector to effect transcription of said desired gene; and

c) an Ig enhancer sequence or a cytomegalovirus enhancer sequence capable of enhancing the expression of said desired gene in said myeloma cell by effecting transcription from said promoter.

2. A process as claimed in claim 1 in which said vector further contains a DNA sequence which causes transcription termination and poly A addition.

3. A process as claimed in claim 2 wherein said sequence is an SV40 poly A signal sequence or an Ig poly A signal sequence.

4. A process as claimed in claim 1, 2 or 3 wherein said enhancer is positioned in said vector upstream from said promoter and within 1000 base pairs of said promoter.

5. A process as claimed in any one of claims 1 to 4, wherein said vector contains Ig gene untranslated regions and said enchancer is positioned within one of said regions.

6. A process as claimed in any one of claims 1 to 5, wherein said protein is t-PA, hepatitis B surface antigen, or a fertility hormone.

7. A process as claimed in any one of claims 1 to 6, wherein said promoter sequence is an Ig light chain promoter, and said Ig enhancer is an Ig heavy chain enhancer.

8. A process as claimed in any one of claims 1 to 7, wherein said promoter sequence is an Ig kappa light chain promoter.

9. A process as claimed in claim 7 or 8, wherein said vector further comprises an Ig poly A signal sequence.

10. A method for producing a desired non-Ig protein in a myeloma cell, comprising culturing a myeloma cell preparable by a process as claimed in any one of claims 1 to 9 and isolating said desired non-Ig protein from said cell or its medium.

11. A process for preparing a vector comprising ligating

a) a gene encoding a desired non-Ig protein;

b) a promoter sequence chosen from the promoter sequence of an Ig gene, a mammalian metallothionein gene, or a cytomegalovirus, positioned in said vector to effect transcription of said desired gene; and

c) an Ig enhancer sequence or a cytomegalovirus enhancer sequence capable of enhancing the expression of said desired gene in a myeloma cell by effecting transcription from said promoter.

FIG 1

FIG 2a

FIG 2b

FIG 3a

FIG 3b

RI

Ig Promoter

Xho I

Pvu II

plasmid
C

Sal I

neo

Light Chain
Enhancer

SV40 Poly A

Bam

1. Digest with Bam HI/Sal I
2. Ligate Sal I Linkers

RI

Ig Promoter

pBR322

Xho I

Sal I

SV40 Poly A

neo

**FIG 3c**

PvuII

1. Eco RI Digest
2. Ligate Xba I Linkers
3. Ligate heavy chain enhancer in
   Xba I fragment from $pV_HC_K$

Xba I   Heavy Chain Enhancer

pBR322

Ig Promoter

Xho I

plasmid
D

Sal I

SV40 Poly A

neo

PvuII

# FIG 3d

**pLT31X** (circular plasmid map)
- pBR322
- RI
- Xho I
- Pvu II
- neo
- SV40 Poly A
- Hpa I
- Bam
- Hpa I
- Ig Poly A

1. Hpa I Digest (partial)
2. Isolate linears
3. Sal I linker ligation

(circular plasmid map)
- pBR322
- RI
- Xho I
- Pvu II
- neo
- SV40 Poly A
- Hpa I
- Bam
- Sal I

1. Sal I/ Hpa I Digest
2. Isolate small fragment

Hpa I ▨ ━━━━━━ ■ Sal I
Bam

**plasmid D** (circular plasmid map)
- pBR322
- Xba I
- Xho I
- Sal I
- Hpa I
- SV40 Poly A
- neo
- PvuII

1. Sal I/Hpa I Digest
2. Isolate large fragment

Ligate

**plasmid E** (circular plasmid map)
- Xba I
- Heavy Chain Enhancer
- Ig Promoter
- Xho I
- Sal I
- pBR322
- PvuII
- neo
- SV40 Poly A
- Hpa I
- Bam
- Ig Poly A

FIG 4a

FIG 4b

FIG 4c

FIG 4d

FIG 5

FIG 6

0 255 320

FIG 7